# FASCICULE DE BREVET EUROPEEN

(11) **EP 3 280 258 B1**
(45) Date de publication et mention de la délivrance du brevet: **10.07.2019**
(21) Numéro de dépôt: 16738475.9
(22) Date de dépôt: 08.04.2016
(51) Int. Cl.: A01M 1/20

(54) **SYSTÈME MATRICIEL AUTONOME PILOTÉ POUR LA DISTRIBUTION CONTROLÉE DE SUBSTANCES ACTIVES**
GESTEUERTES EIGENSTÄNDIGES MATRIXSYSTEM ZUR GESTEUERTEN VERTEILUNG VON WIRKSTOFFEN
CONTROLLED STAND-ALONE MATRIX SYSTEM FOR THE CONTROLLED DISTRIBUTION OF ACTIVE SUBSTANCES

(30) Priorité: 10.04.2015 FR 1500747
(43) Date de publication de la demande: 14.02.2018
(73) Titulaire: AB7 Innovation S.A.S.U., 31450 Deyme (FR)
(72) Inventeur: CHELLE, René, 31450 Deyme (FR); NGUYEN, David, 31400 Toulouse (FR); VILBERT, Arnaud, 31450 Baziège (FR); AUGIER, Sylvain, 31400 Toulouse (FR)
(86) Numéro de dépôt international: PCT/FR2016/000071
(87) Numéro de publication internationale: WO 2016/162608

(56) Documents cités:
- EP-A1- 1 579 762
- WO-A1-2005/008352
- FR-A1- 2 322 546
- FR-A1- 2 722 368
- US-A1- 2003 049 025

## Description

La présente invention se situe dans le domaine des systèmes permettant de distribuer des substances actives pour traiter et/ou contrôler des cibles.

Précisément, la présente invention a trait à un système matriciel piloté pour la distribution de substances actives en quantité contrôlée et uniquement à des moments opportuns, pour que la quantité relarguée réponde parfaitement à un besoin d'efficacité préalablement établi et/ou connu en fonction de la cible visée.

On connaît des dispositifs destinés à diffuser des substances actives telles que des phéromones, des insecticides, du parfum, des désodorisants. Généralement, on peut les classer en trois grandes catégories :
- dispositif réservoir pour lequel la substance active en solution liquide est chargée dans un réservoir. Ladite substance se diffuse dans l'atmosphère soit par migration à travers la paroi en polymères semi-perméables dudit réservoir soit par pulvérisation sous forme de gouttelettes.
- dispositif matriciel dans lequel la substance active solubilisée dans un solvant compatible est incorporée dans une matrice polymère de formes diverses.
- dispositif à microparticules dans lequel la substance active est préalablement chargée dans un micro-support absorbant pour une application par pulvérisation après dispersion dans un liquide vecteur.

La présente invention se classe dans la catégorie des dispositifs matriciels associés à d'autres composants pour que l'ensemble constitue un système.

Plus particulièrement, la présente invention a pour objet un système matriciel autonome piloté pour la distribution à vitesse contrôlée et à des moments opportuns et précis de substances actives incorporées dans une matrice polymérique. Ledit système comprend au moins trois composants essentiels :
- une matrice polymérique chargée en composition contenant au moins une substance active ;
- un élément chauffant encastré dans ladite matrice chargée, ledit élément étant apte à chauffer de manière homogène ladite matrice pour enclencher la libération de ladite substance active, et
- un générateur de courant électrique alimentant une carte électronique de commande logée dans un boitier électronique.

On connaît aussi des dispositifs en matrice polymère hydrophobe pour la distribution de substances actives. Lesdits dispositifs peuvent être notamment en forme de bracelets, de colliers, de plaquettes ou toute autre forme. De tels dispositifs permettent uniquement d'obtenir une diffusion passive à la température ambiante en continue de substances actives qui y sont incorporées. Cette diffusion démarre dès l'ouverture de l'emballage et ne peut être stoppée sauf à être introduite dans un nouvel emballage étanche, ce qui oblige l'utilisateur à des manipulations supplémentaires. Le choix des vecteurs de formulation desdites substances actives, celui des polymères pour la matrice ainsi que les dimensions du dispositif final constituent l'unique façon de contrôler le relargage desdites substances. Or, la diffusion continue n'est pas toujours adaptée à certains traitements bien spécifiques, par exemple lorsque l'on souhaite obtenir une diffusion localisée d'actif uniquement à des moments précis. De plus, certaines matrices polymériques à diffusion passive à la température ambiante, séquestrent une quantité non négligeable, allant jusqu'à 50% en poids de substance active à la fin de son utilisation présumée. En effet, la Demanderesse a observé que certaines molécules actives non volatiles ou faiblement volatiles restent toutefois séquestrées dans la matrice malgré leur formulation optimale au moyen de bons vecteurs. Par ailleurs certains dispositifs médicamenteux requièrent que l'on n'utilise qu'une partie du potentiel complet de relargage afin d'être sûr d'utiliser une zone de la cinétique où la dose est constante et à un niveau au dessus du seuil d'efficacité. Ce mode de fonctionnement entraîne un gaspillage important des substances actives et la mise en déchet de ces dispositifs ne sont pas épurés de leurs substances actives.

Bien que la Demanderesse ait déjà développé des dispositifs en matrice polymérique à diffusion passive en forme de plaquettes ou de bracelets tels que décrits dans les brevets FR2901096, FR2951645 et FR2959396, aucun dispositif connu basé sur une matrice polymère ne permet de délivrer tout type de substances actives à une vitesse contrôlée uniquement aux moments opportuns et précis. Par ailleurs, aucun dispositif matriciel connu ne parvient à relarguer les substances actives à vitesse contrôlée pour que la quantité relarguée réponde parfaitement à un besoin d'efficacité préalablement établi et/ou connu.

Afin d'apporter une amélioration au fonctionnement des dispositifs matriciels à diffusion passive, d'autres composants ont été ajoutés pour que l'ensemble constitue un système. Parmi les composants ajoutés, on peut citer des éléments chauffants de type plaque ou résistance chauffante appliqués à la matrice polymérique. A ce sujet, la technique antérieure distingue deux catégories de système matriciel bien distinctes :
- soit, lesdits éléments chauffants sont appliqués à des matrices en polymères hydrophiles d'origine végétale chargées d'actif,
- soit, lesdits éléments chauffants sont associés à des matrices en polymères hydrophobes mais celles-ci ne sont pas chargées d'actif.

On connaît donc des systèmes matriciels comprenant des éléments chauffants pour promouvoir la diffusion de substances actives lesquelles sont volatiles dans la quasi-totalité des cas.

Par exemple, le brevet FR2722368 enseigne sur un diffuseur à mèche d'insecticides muni d'une batterie pour une diffusion aérienne par volatilité durant environ 12 heures. Le diffuseur comprend une plaque rayonnante sur laquelle est placé un tampon en matrice cellulosique imprégnée d'insecticide de type pyréthroïdes. La surface de ladite plaque est chauffée à une température inférieure à 130°C pour préserver l'insecticide. L'élément chauffant consiste en une thermistance organique constituée par un mélange d'une résine de polyéthylène ou une résine de polypropylène et de carbone. Mais, la matrice utilisée est de nature hydrophile et aucune carte de pilotage électronique préalablement programmée n'est présente dans le diffuseur pour piloter la batterie afin d'obtenir une quantité contrôlée d'insecticides pour une meilleure efficacité.

Dans le même esprit, les brevets FR2431256, EP1108358, EP1101500, EP1579762, EP1372161, WO2005/008352 et FR2322546 décrivent un système comprenant un élément chauffant et une mèche hydrophile plongée dans un réservoir contenant une composition liquide d'insecticide et/ou de parfum pour une diffusion aérienne par volatilité.

Le brevet BE1001662 décrit un dispositif pour la diffusion aérienne par volatilité d'insecticides et de désodorisants comprenant des éléments chauffants électriques en plaque céramique à l'intérieur de laquelle est montée une résistance électrique, ladite plaque étant destinée à supporter et à chauffer une plaquette chargée d'insecticides. Pour faire fonctionner le dispositif, on dépose ladite plaquette sur la plaque puis on met en marche le montage électrique. Mais, le document est muet sur la nature physico-chimique de la matrice et aucune carte de pilotage électronique préalablement programmée n'est présente afin d'obtenir une quantité efficace d'insecticides.

La demande de brevet US2007/257016 revendique un dispositif à batterie électrique pour la diffusion de substance insecticide volatile sous forme de nuage de vapeur. Ladite substance insecticide est imprégnée dans une matrice poreuse ou fibreuse telles que les fibres tissées ou non tissées. Une résistance électrique disposée à l'extérieure de ladite matrice est mise en contact avec elle pour provoquer la diffusion de l'insecticide. Dans le même esprit, le brevet US6309986 décrit un système comprenant une matrice cellulosique stratifiée imprégnée d'insecticide volatile. Une résistance chauffante métallique est disposée sur la couche contenant l'insecticide de manière à chauffer celle-ci et provoquer la diffusion de l'insecticide. Mais, la matrice est hydrophile et la batterie n'est nullement pilotée par aucune carte électronique préalablement programmée. De plus, seules les substances volatiles sont compatibles avec ces deux systèmes.

Quant aux matrices hydrophobes non chargées de substance active, on peut citer le brevet FR2888715 qui décrit un dispositif destiné à protéger les plantations contre le gel, ledit dispositif étant sous forme de film plastique multicouche dont au moins une des couches comporte des éléments chauffants. Lesdits éléments chauffants sont répartis en modules alimentés par une source électrique. Mais, le film multicouche du système n'est chargé d'aucune substance active. Dans le même registre, la demande internationale WO2005039240 divulgue un dispositif en film en polymères thermoplastiques multicouche répondant à des besoins en chauffage sur de grandes surfaces, notamment en viticulture. Ledit dispositif comporte des moyens conducteurs électriques pour pouvoir provoquer par effet Joule un échauffement à la surface dudit film. Mais, le film multicouche du système n'est chargé d'aucune substance active.

Dans une conception plus complexe, le brevet EP1629696 divulgue un réchauffeur de vêtement flexible comprenant des couches laminées dont au moins une d'entre elles comporte des éléments chauffants. Lesdits éléments chauffants sont connectés à une batterie et sont intégrés dans le tissu dont certaines fibres sont métallisées. Une des couches est un film adhésif en polymères thermoplastiques imperméables. Des capsules contenant des substances actives volatiles sont fixées sur les fibres. Lesdites substances volatiles sont libérées par éclatement des capsules sous l'effet de la chaleur. Mais, la matrice est stratifiée et les substances actives doivent être encapsulées. Par ailleurs, lesdites substances ne sont pas incorporées directement dans la couche en polymères thermoplastiques et aucune carte de pilotage électronique n'est présente pour piloter la batterie.

Malgré toutes les solutions proposées par la technique antérieure, il existe un besoin de fournir un système matriciel autonome piloté pour la distribution contrôlée de tout type de substances actives à savoir liquides et solides, volatiles ou non volatiles. Ledit système permet de libérer essentiellement sur commande lesdites substances actives incorporées dans une matrice polymère par chauffage à des moments opportuns et précis en quantité suffisante pour conférer à un traitement donné une meilleure efficacité vis-à-vis d'une cible visée. La présente invention a pour but d'obvier aux inconvénients rencontrés dans l'art antérieur.

Un objectif de la présente invention est de fournir un système matriciel autonome piloté par une carte électronique programmée pour la distribution contrôlée et modulable d'au moins une substance active en quantité suffisante préalablement déterminée uniquement à des moments opportuns et précis afin de conférer à un traitement une meilleure efficacité en fonction de la cible visée, laquelle étant constituée par des insectes ou un environnement ouvert notamment un champ, ou fermé notamment un local ou une habitation.

Un autre objectif de la présente invention est de proposer un système matriciel apte à distribuer au moins une substance active en quantité bien déterminée tout en minimisant la diffusion passive à la température ambiante de ladite substance active depuis la matrice polymère mais en optimisant la libération de la substance active uniquement à des moments opportuns et précis.

Un autre objectif de la présente invention est de proposer un système au sein duquel la matrice polymérique libère la quasi-totalité de la substance active qu'elle contenait à la fin de l'utilisation du système à l'inverse d'un dispositif en matrice polymère à diffusion passive qui garde séquestrée plus de 10% de ladite substance à la fin de son utilisation. Cela permet d'exploiter la totalité de la capacité réservoir qu'offre la matrice pour des durées d'utilisation allongée.

Un autre objectif de la présente invention est de fournir un système matriciel autonome piloté pour la distribution contrôlée de substances actives non volatiles à la température ambiante.

Encore un autre objectif de la présente invention est de fournir un système matriciel autonome piloté pour la distribution contrôlée et simultanée ou décalée de plusieurs substances actives identiques et/ou différentes, les matrices multiples étant chargées de substances actives et peuvent être montées en série, chacune des matrices étant chargée en substance active identique ou différente.

Encore un autre objectif de la présente invention est de fournir un procédé qui utilise le système matriciel selon la revendication 1 pour traiter efficacement une cible se trouvant au même endroit ou à des endroits spatialement différents uniquement à des moments opportuns et précis par diffusion séquentielle ou en continue d'une quantité suffisante préalablement déterminée d'au moins une substance active.

Au sens de l'invention, on entend par « boitier électronique de commande » un ensemble comprenant au moins une carte électronique de commande programmée en tenant compte d'au moins trois paramètres essentiels que sont l'intensité de chauffe, la durée de chauffe et la fréquence de chauffe. La programmation de la carte est adaptée pour qu'elle soit capable de gérer simultanément au moins ces trois paramètres en fonction de la cible visée. Grâce à la programmation, le système matriciel distribue la substance active selon un profil de relargage de référence. La mise en marche du système se fait de manière automatique ou au moyen des enclencheurs embarqués ou manuels comme les interrupteurs et analogues. Avantageusement, afin d'optimiser et de compléter le relargage de référence, ladite carte est associée à au moins un collecteur de signaux, ledit collecteur pouvant être autonome. Ainsi, ladite carte pilote la commande de la mise en marche ou l'arrêt de l'alimentation en courant électrique de l'élément chauffant par le générateur, en fonction du signal envoyé par au moins un collecteur au moyen d'un réseau de communication filaire et/ou sans fil (exemple : type Wi-Fi®, type Bluetooth ou type GSM), selon une structure de communication entre collecteurs, réseau et stations, communément appelée *Wireless Body Area Network* (WBAN). Les stations, fixes de type ordinateurs ou, mobiles de type téléphones et analogues, sont dotées d'une interface logicielle permettant la reconnaissance de chacun des collecteurs au réseau filaire et/ou sans fil ainsi que les signaux qu'ils émettent. De même, le pilotage de l'alimentation en courant électrique par ladite carte peut s'effectuer au moyen du réseau sans fil cité ci-dessus. Dans ce cas, il est prévu de disposer des émetteurs et des récepteurs respectivement sur le collecteur et sur ladite carte. Le collecteur de signaux est un dispositif apte à transformer les grandeurs physico-chimiques et physiologiques en signaux électriques. Lesdites grandeurs sont par exemple la température, la pression, le pH, l'air, l'humidité, la luminosité, la présence, le mouvement, la fréquence cardiaque, la glycémie, la pression vasculaire ou encore le rythme de la respiration. Ledit collecteur peut être par exemple un capteur, une sonde, un détecteur ou l'association de ceux-ci. Plus clairement, la carte électronique comporte, notamment, un processeur de calcul pour élaborer la commande d'activation de l'élément chauffant, une unité de mémoire destinée à stocker les données récoltées par des différents collecteurs répartis, autonomes et communicants, pour leur fusion par un algorithme afin de définir un profil de relargage type, adapté à un traitement, en fonction des besoins de la cible. Ladite carte fait partie intégrante d'une architecture électronique, embarquée ou non, permettant la réception, l'enregistrement, la surveillance et le traitement des signaux en provenance de tous les collecteurs du système.

Ainsi, la carte électronique de commande pilote le système conforme à l'invention en exécutant les fonctions choisies parmi :
- gestion de la mise en marche et de l'arrêt de l'alimentation en courant électrique de(s) l'élément(s) chauffant(s) par le générateur ;
- réglage de la puissance développée par ledit générateur ;
- réglage la durée, l'intensité et la fréquence de la chauffe ;
- gestion des séquences d'activation ;
- gestion de la durée totale d'un traitement ou d'une application ;
- gestion, le cas échéant, de la mise en marche, l'arrêt et la puissance d'un ventilateur.

On entend par « matrice active » une matrice polymère dans laquelle est incorporé tout type de composition active liquide ou solide, volatile ou non volatile. A la fin de l'incorporation, ladite matrice active est sèche.

On entend par une distribution « contrôlée et modulable » un mode de relargage de la substance active depuis la matrice se faisant de manière continue et/ou séquentielle. Une distribution séquentielle peut être représentée par un diagramme en dents de scie, ou par un diagramme de pulsation dans lequel les amplitudes et/ou les fréquences sont variables et dépendent des trois paramètres essentiels de la programmation cités ci-dessus.

On entend par une quantité relarguée de substance active « minimisée » une quantité de substance active libérée, à la température ambiante par diffusion passive par la matrice active au repos à un taux inférieur à 10%, préférentiellement entre 0,1% et 5% en poids par rapport à la quantité totale initialement incorporée pendant une durée de 10 jours.

On entend par une substance active libérée dans sa « quasi-totalité » le fait qu'à la fin de l'utilisation du système, la matrice active a relargué au minimum 90% de ladite substance par rapport à la quantité totale initialement incorporée.

Ainsi, la présente invention a pour objet un système matriciel autonome piloté pour la distribution contrôlée et modulable uniquement à des moments opportuns et précis d'au moins une substance active, ledit système comprenant :
- une matrice polymérique non stratifiée chargée en ladite substance active, ladite matrice comportant au moins une sonde thermique,
- au moins un élément chauffant encastré dans la masse de ladite matrice chargée, ledit élément étant apte à chauffer de façon homogène l'intégralité de ladite matrice de manière à dilater temporairement le réseau polymérique pour enclencher le relargage de ladite substance active,
- un générateur de courant électrique relié à une carte électronique de commande, elle-même reliée audit élément chauffant, dont l'alimentation en courant électrique par ledit générateur est pilotée par ladite carte logée dans un boitier électronique, ladite carte étant reliée, en outre, à ladite au moins sonde thermique.

De manière surprenante, connaissant les effets de l'action de la chaleur sur les matrices actives, les recherches menées par les inventeurs ont démontré que sur certaines catégories de polymères, une chaleur quantitativement bien dosée et distribuée de manière homogène au sein d'une matrice active permet de déstructurer temporairement le réseau polymérique afin d'accélérer le relargage de tout type de substances actives solide et liquide. La température intra-matricielle atteinte au cours de la chauffe ne doit pas être supérieure à la température de dénaturation de la substance active incorporée. Ce constat est mis à profit par la Demanderesse qui l'a associé avec une carte électronique de commande préalablement programmée pour obtenir un relargage en quantité déterminée et suffisante de substance active conférant à un traitement donné une meilleure efficacité en fonction de la cible visée. Le profil de relargage contrôlé de la substance active obtenu grâce à la programmation de ladite carte constitue le profil de référence de base.

Conformément à l'invention, afin d'obtenir un chauffage homogène de ladite matrice, l'élément chauffant est encastré dans la matrice active. L'élément chauffant peut aussi être surmoulé directement dans ladite matrice, il peut également s'emboiter avec celle-ci ; dans ce dernier cas, il peut être amovible.

Etant donné que la dilatation temporaire du réseau polymérique et l'augmentation de la tension de vapeur de la substance active elle-même favorisent son relargage à partir de la matrice active, la Demanderesse a déterminé que l'encastrement d'au moins un élément chauffant dans la matrice active constitue un moyen efficient pour assurer un chauffage intra-matriciel homogène de sorte que le chauffage par effet Joule entraîne la libération de la substance active. Le nombre d'éléments chauffants devant être présents ou la géométrie dudit élément chauffant tient compte des dimensions de la matrice active à chauffer, ainsi que des propriétés physico-chimiques des substances actives à savoir les températures de dénaturation et d'ébullition, des propriétés thermiques de la matrice à savoir leur conductivité thermique et les températures de dégradation et de ramollissement des polymères. Afin de favoriser l'accélération du relargage de la substance active, on privilégie une forme géométrique ajourée ou toute autre conception augmentant la surface d'échange de la matrice.

Conformément à l'invention, le générateur de courant électrique est nécessairement connecté à la carte électronique de commande qui pilote l'alimentation en courant électrique par ledit générateur de l'élément chauffant, en plus des autres composants électriques du système, comprenant notamment le(s) ventilateur(s) et les collecteurs ; ladite carte étant préalablement programmée. La programmation consiste à faire varier, de manière dépendante ou non les uns par rapport aux autres, trois paramètres essentiels que sont l'intensité de chauffe, la durée de chauffe et la fréquence de chauffe en fonction de la composition de la matrice, de la propriété physico-chimique de la substance active et surtout de la quantité nécessaire et suffisante devant être relarguée pour conférer à un traitement donné une meilleure efficacité. L'intensité de la chauffe est directement proportionnelle à la puissance du générateur électrique. La quantité de substance active nécessaire et suffisante est préalablement déterminée et connue par exemple par des expériences, des tests en laboratoires, des produits existants disponibles sur le marché unanimement reconnus ou encore par la littérature. Ainsi, une fois qu'il est fait rapport d'un changement significatif du comportement, un changement physiologique significatif, ou un changement environnemental significatif en comparaison avec des valeurs de références, la fonction de commande de la carte électronique décidera automatiquement, selon des prescriptions définies par l'algorithme, de déclencher l'activation de l'élément chauffant, de la fréquence d'activation et de la durée d'activation, ces trois paramètres étant liés ou non entre eux.

De fait, ladite carte pilote l'activation de l'élément chauffant en alimentant ce dernier en courant électrique entrainant le chauffage de la matrice, ce qui enclenche le relargage à vitesse contrôlée et modulable de la substance active. La chauffe s'effectue de façon répétitive ou séquentielle de sorte que la matrice relargue la quasi totalité de la substance active. A la fin de chaque chauffe, il est parfois privilégié de laisser la matrice reprendre son comportement initial en la laissant se refroidir avant de la réactiver avec une autre chauffe. Dans certain cas, afin d'obtenir un relargage de substance active de manière continue, il est privilégié de réactiver de manière successive et séquentielle la matrice par chauffage sans la laisser se refroidir jusqu'à la température ambiante.

Conformément à l'invention, la carte électronique de commande est programmée pour activer la mise en marche et l'arrêt ainsi que de régler la puissance du générateur électrique. La chauffe est systématiquement coupée soit lorsque la température intra-matricielle relevée par des sondes thermiques avoisine de 2°C à 5°C en dessous de la température de dénaturation de la substance active ou celle de la matrice, soit lorsqu'elle est égale à la température de consigne préalablement indiquée lors de la programmation.

Selon un mode de réalisation de l'invention, la durée d'une chauffe est adaptée en fonction des dimensions, des caractéristiques thermiques à savoir la conductivité thermique de la matrice ainsi que des propriétés physico-chimiques à savoir la température de dégradation ou de dénaturation de la substance active et surtout de la dose indispensable à relarguer par rapport à la cible dans un milieu considéré. La durée d'une chauffe peut varier par exemple entre 3 secondes et 60 minutes voire plusieurs heures.

Selon un mode de réalisation de l'invention, ledit générateur est apte à développer une puissance électrique suffisante pour élever la température de la matrice à la température programmée. La puissance électrique devant être reçue par la matrice active tient compte de la surface de ladite matrice à chauffer ainsi que des dimensions de celle-ci. Par exemple, une puissance d'environ 60 watts permet de porter à 70°C en 15 minutes une pièce de polymère d'un volume d'environ 600 cm³. Ledit générateur électrique est choisi parmi une batterie d'accumulateurs, une pile électrique, une pile à combustible, un groupe électrogène; un panneau solaire ou le réseau électrique public connu de l'homme du métier.

Selon un autre mode de réalisation de l'invention, la carte électronique est apte à gérer, le cas échéant, la mise en marche, l'arrêt et la puissance d'un ventilateur. Ledit ventilateur est placé à proximité de la matrice active de manière à favoriser la propagation de la substance active dans l'atmosphère et à générer un gradient de concentration qui peut être indispensable dans le contrôle de certains nuisibles.

Selon un mode de réalisation de l'invention, afin de compléter et d'optimiser le relargage de référence, la carte électronique est reliée à un collecteur de signaux via un réseau filaire et/ou sans fil. Ledit collecteur est un dispositif apte à transformer les grandeurs physico-chimiques et physiologiques en signaux électriques. Lesdites grandeurs sont par exemple la température, la pression, le pH, l'air, la fumée, l'humidité, le bruit, la luminosité, la présence, le mouvement ou l'association de celles-ci. Ledit collecteur peut être par exemple un capteur, une sonde, un détecteur ou l'association de ceux-ci. En fonction des signaux, ladite carte adapte la commande du générateur et donc les conditions d'activation des éléments chauffants.

Les collecteurs ci-dessus décrits sont reliés au boitier électronique de commande au moyen d'un réseau de communication filaire et/ou sans fil et sont placés sur la matrice active, dans la matrice active, à proximité de la matrice active, dans l'environnement qui l'entoure ou l'association de ceux-ci.

Conformément à l'invention, l'élément chauffant est une résistance chauffante électrique choisi parmi un fil chauffant gainé, un fil résistif en alliage nickel-chrome ou en alliage de titane, une résistance en forme de plaque, de bille métallique, ou de crayon chauffant ou l'association de ceux-ci. Dans le cas d'un fil chauffant, la résistivité est par exemple comprise entre 1 et 100 Ω.

Selon un mode réalisation de l'invention, la matrice polymère elle-même peut être modifiée de façon à améliorer sa conductivité thermique. Pour ce faire, il est adjoint de particules métalliques fines à la matrice polymère, qui influent significativement sur la conductivité thermique de celle-ci afin de limiter son inertie thermique et améliorer la rapidité de diffusion. Comme particules conductrices thermiques, on peut citer :
- les polymères additivés de charges conductrices dérivées du carbone tels que le noir, les fibres ou tubes de carbone, des particules de graphites,
- les métaux tels que l'aluminium, le cuivre des pigments métalliques tels que l'argent, le nickel ou le cuivre, ou des alliages tels que fer/cuire, aluminium/cuivre et autres associations connues de l'homme du métier.
- les céramiques conductrices.

Dans le cas d'un élément chauffant sous forme de plaque, celle-ci est composée d'un matériau très conducteur thermique apte à supporter une puissance maximale par exemple jusqu'à 15 watts/cm².

Selon un mode de réalisation de l'invention, afin d'optimiser la diffusion de la température générée par le chauffage au sein de la matrice active, des dissipateurs thermiques tels que des substrats métalliques isolés en plaques d'aluminium surmontées d'une fine couche isolante en céramique, elle-même surmontée d'un circuit imprimé en cuivre sont associés aux éléments chauffants. Les dissipateurs sont directement reliés au générateur électrique. Lesdits dissipateurs remplissant les caractéristiques ci-dessus énumérées sont des dissipateurs conventionnels bien connus de l'homme du métier tels que les dissipateurs en substrat métallique isolé (SMI). Lesdits dissipateurs sont associés, par exemple par soudure, auxdits éléments chauffants.

Le chauffage par effet Joule généré par l'élément chauffant est apte à provoquer la libération de la substance active incorporée dans la matrice active sans induire ni dégradations chimiques ni dégradations structurelles irréversibles de ladite matrice. Dans tous les cas, lorsque la matrice active est à base de polymères thermodurcissables, la température intra-matricielle atteinte lors de la chauffe est inférieure à la température de dégradation de desdits polymères thermodurcissables. Ladite température de dégradation est inférieure ou égalé à 200°C. Lorsque la matrice active est en polymères thermoplastiques, la température intra-matricielle atteinte lors de la chauffe est inférieure au point de ramollissement desdits polymères thermoplastiques. Ladite température est inférieure ou égale à 180°C.

Conformément à l'invention, la substance active représente entre 0,1% et 50% en poids par rapport au poids total de la matrice active. La substance active est préférentiellement formulée en la dispersant dans un vecteur et/ou solvant compatible connu de l'homme du métier. D'autres additifs tels que des plastifiants, des catalyseurs, des tensioactifs, des stabilisants, des agents amérisants, des colorants, des charges diverses peuvent être ajoutés lors de la formulation de ladite composition liquide. Lesdits additifs sont ceux couramment utilisés par l'homme du métier. La substance active peut être liquide ou solide (amorphe et cristalline). Les substances actives ont des fonctions telles qu'attractive comme des kairomones, répulsive, insecticide, insectifuge, fongicide, larvicide, bactéricide, rodenticide, parfumante, désodorisante, cosmétique, ou phytosanitaire.

Les substances insecticides/insectifuges sont choisies parmi les composés de la famille des pyréthrinoides et leurs dérivés, les pyréthrines, le DEET (N, N-diéthyl-3-méthylbenzamide), l'Icaridine®, l'IR3535® (éthyl-3-acetyl(butyl)amino-propanoate), les organochlorés, les organophosphorés, les organophosphates (ex : le Diazinon®, le Chlorpyrifos®), les carbamates (ex : le Propoxur®, le Fipronil®), les néonicotinoïdes, les sulfones et sulfonates, les formamidines, les benzoylurées, les huiles essentielles, les extraits végétaux, les hydrocarbures terpéniques et leurs dérivés, les huiles végétales ou le mélange de ceux-ci.

A titre d'exemple de substances actives non volatiles ou faiblement volatiles, on peut citer le DEET, l'IR3535®, l'Icaridine®, le citronellol, le citriodiol dont les températures d'ébullition sont respectivement de 240°C, 292°C, 272°C, 225°C, 268°C.

Les substances attractives sont choisies parmi des molécules sémiochimiques, des huiles essentielles, des extraits végétaux, des huiles végétales et des arômes.

Les substances désodorisantes sont des molécules d'origine naturelle ou de synthèse choisies parmi les huiles essentielle, des essences de parfum, des parfums, des extraits végétaux.

Dans le cas des polymères thermoplastiques, la substance active est formulée en composition liquide grâce au concours des vecteurs liquides compatibles pour obtenir une composition active liquide homogène. Ensuite, ladite composition liquide est incorporée dans des granulés polymères selon les enseignements des brevets FR2901172, FR2956345 et la demande FR14/02377 déposés au nom de la Demanderesse, incorporés par référence dans la présente. Les vecteurs liquides compatibles employés dans les procédés cités ci-dessus sont ceux qui sont couramment utilisés et connus de l'homme du métier.

Dans le cas des polymères thermodurcissables, la substance active formulée en composition liquide de la même manière que pour les polymères thermoplastiques. Ladite composition active liquide est ensuite mélangée à la température ambiante avec la solution liquide du complexe de phase polyol selon les enseignements de la demande de brevet FR2992325 déposée au nom de la Demanderesse, incorporée par référence dans la présente.

Conformément à l'invention, la matrice est fabriquée à partir de polymères thermoplastiques synthétiques ou biousourcés, biodégradables ou non, ou de polymères thermodurcissables.

Les polymères thermoplastiques sont d'origine fossile. Ils sont choisis parmi les copolymères d'éthylène et d'acétate de vinyle dont le taux d'acétate de vinyle est compris entre 15% et 60%, les élastomères de polyuréthanes thermoplastiques (TPU) à base de polyester ou de polyéther, les copolyamides et les polyamides (PA) de grades absorbants tels que PA6, PA10, PA12, les polyéthers block amides de grades absorbants, les polyéthylènes de basse densité (PEBD) de grades absorbants, les polyéthylènes greffés amidon, les polychlorures de vinyle et ses copolymères, les polyesters, les polymères styréniques tels que les SEBS, SIS ou un mélange de ceux-ci.

Les polymères biodégradables sont des biopolymères extraits directement de la biomasse et leurs dérivés, ou les polyesters obtenus par fermentation bactérienne ou par polymérisation de monomères issus de la biomasse, ou des ressources fossiles, ainsi que l'association de ces trois groupes. On peut citer les dérivés les biopolymères comme l'acétate de cellulose, les polyesters comme le polybutylène-adipate-co-téréphtalate. On peut également utiliser l'association de l'acide polylactique et du polyhydroxybutyrate ou encore l'association d'un agro-polymère et d'un polyester comme l'amidon et du polybutylène-adipate-co-téréphtalate, celle de l'amidon de maïs et de l'acide polylactique ou un mélange de tous les polymères biodégradables ci-dessus énumérés.

Les polymères thermodurcissables sont des polyuréthanes réticulés obtenu à partir d'une résine polyol liquide ou rendu liquide et un isocyanate liquide ou rendu liquide puis mis en forme par coulée. Avantageusement, le choix des deux composants essentiels à savoir la résine polyol et l'isocyanate est adapté en fonction de la propriété mécanique voulue de la matrice ainsi que de son aptitude à minimiser la libération des substances actives incorporées dans la matrice.

L'isocyanate est choisi parmi les isocyanates avec au minimum 2 fonctionnalités, de structure aromatique ou aliphatique. On peut citer les TDI (diisocyanate de toluène), HDI (diisocyanate d'hexaméthylène), MDI (diisocyanate de diphénylméthylène), H12MDI (dicyclohéxylméthane diisocyanate), IPDI (isophorone diisocyanate), NDI (naphtalène diisocyanate), TODI (O-tolidine diisocyanate), PPDI (para-phénylène diisocyanate) et leurs prépolymères.

La résine polyol est choisie parmi celle ayant au minimum 2 fonctionnalités hydroxyle, à chaîne longue ou à chaîne courte, à base de polyesters, de polyéthers, de polythioéthers, de polyacétals, de polycarbonates, de polyesteramides, d'huiles végétales naturellement hydroxylées ou modifiées ou le mélange de ceux-ci. Les polyols peuvent être remplacés par des polyamines ayant au moins 2 fonctionnalités amine, choisies parmi les polyétheramines, aliphatiques ou aromatiques.

Conformément à l'invention, la matrice polymère est mise en forme selon les techniques de plasturgie bien connues de l'homme du métier.

L'autre objet de la présente invention porte sur un procédé pour traiter efficacement des cibles, constituée(s) par des insectes ou une zone délimitée dans un environnement ouvert ou fermé, se trouvant au même endroit ou à des endroits spatialement différents au moyen d'au moins une substance active, dont la diffusion séquentielle ou en continue d'une quantité contrôlée préalablement déterminée uniquement à des moments opportuns et précis, ledit procédé consiste à utiliser le système selon la revendication 1.

Selon un mode de mise en oeuvre, lorsque les matrices actives sont multiples et réparties à différents endroits ou qu'elles sont montées en série, dans un tel cas, d'une part, chaque élément chauffant de chacune des matrices est relié à une carte électronique de commande unique et commune, et est disposée au sein d'un poste central unique faisant office de boitier électronique, à partir duquel ladite carte pilote, de manière individuelle, l'activation de chacun des éléments chauffants, en fonction des données récoltées en provenance des collecteurs et, d'autre part, la carte électronique étant elle-même reliée soit par un câble électrique porteur, lui même relié à un générateur de courant de type secteur, soit reliée à une pile.

En effet, la chauffe est activée de manière séquentielle. Une séquence d'activation étant constituée par la somme de durée de chauffe et la durée du repos de la matrice active, la durée de chauffe est variable, comprise entre 10 secondes et plusieurs heures. Ainsi, le procédé consiste à activer séquentiellement ou en continue la matrice active autant de fois que nécessaire jusqu'à ce que la matrice relargue la quasi totalité de la substance active.

Afin de mieux comprendre l'objet revendiqué, les figures ci-dessous sont destinées à détailler la structure préférée du système tel que décrit dans la présente invention, ainsi que son mode de fonctionnement.
Figure 1 : schéma du système matriciel conforme à l'invention comprenant une matrice active, un élément chauffant relié à un générateur électrique piloté par une carte électronique logée dans un boitier électronique de commande.
Figure 2 : profil de relargage de référence l'huile essentielle de menthe poivrée obtenu par le système matriciel piloté en matrice en polymère thermodurcissable conforme à l'invention.
Figure 3 : profil de relargage de référence de substance très faiblement volatile (citriodiol) obtenu par le système matriciel en matrice en polymère thermoplastique conforme à l'invention.
Figure 4 : profil de relargage de référence de substance non volatile (IR3535®) obtenu par le système matriciel en matrice en polymère thermodurcissable conforme à l'invention.

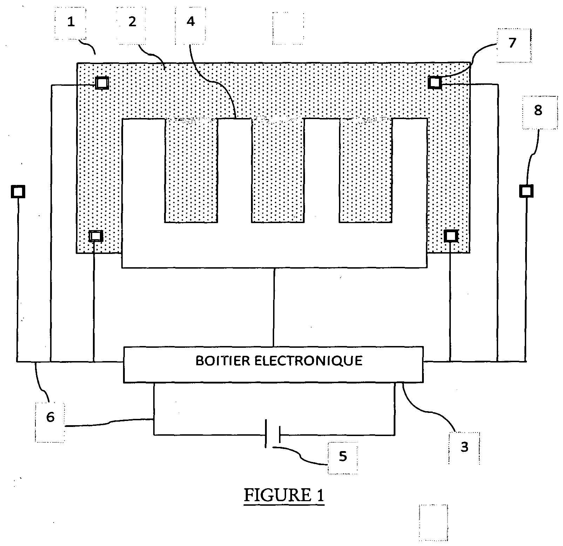

La figure 1 représente le schéma global d'un prototype du système (1) matriciel piloté pour la distribution contrôlée et modulable de substance active conforme à l'invention. Le système (1) est constitué par une matrice active (2) en forme de broche ayant cinq dents. Ladite matrice active (2) peut être de forme cylindrique, polyédrique, ou sphérique. L'élément chauffant (4) également en forme de broche ayant quatre dents est encastré dans la matrice active (2) de manière à chauffer uniformément par effet Joule ladite matrice (2). Selon une variante de réalisation de l'invention, afin d'optimiser la dissipation de la chaleur générée par l'élément chauffant (4) à l'intérieur de la matrice (2), un dissipateur thermique (non représenté) peut être associé audit élément chauffant (4). Dans ce cas, le dissipateur est directement relié au générateur (5) ou associé à l'élément chauffant (4) par soudure.

L'élément chauffant (4) est relié au boitier électronique de commande (3) dans lequel est logée une carte électronique (non représentée) préalablement programmée en termes de durée de chauffe, d'intensité de chauffe et de fréquence de chauffe pour le piloter afin d'obtenir un profil de relargage de référence de substance active. Ledit boitier électronique de commande (3) est relié à un générateur électrique (5) au moyen d'un réseau filaire (6). Afin d'optimiser le relargage de référence, des sondes thermiques (7) placées sur la matrice (2) relèvent la température intra-matricielle. De la même manière, la carte électronique adapte l'activation de l'élément chauffant (4) en recevant les signaux transmis par les collecteurs (8) au boitier électronique (3). C'est par exemple le cas de matrices actives (2), multiples ou montées en série, et placées individuellement à des endroits spatialement différents où les collecteurs (8) sont des détecteurs de mouvement. La chauffe s'arrête lorsque la température relevée par au moins un des capteurs thermiques (7) est proche, entre 2°C à 5°C en dessous de la température de dénaturation de la substance active ou de la température de consigne préalablement fixée. Un ou plusieurs éléments chauffants (4) sont encastrés dans la matrice active (2) pour la chauffer de manière homogène. Le boitier (3) est relié au générateur (5) au moyen d'un réseau filaire (6) conventionnel.

Selon une variante de réalisation de l'invention, le système matriciel (1) est monté en série. Dans ce cas, le boitier électronique de commande (3) est relié simultanément à plusieurs éléments chauffants (2) différents encastrés dans des matrices actives (2) différentes. Des interrupteurs électriques peuvent alors être placés au sein du montage électrique reliant ledit boitier électronique (3) et chacun des éléments chauffants (2). De cette manière, la chauffe des matrices actives (2) montées également en série, est contrôlée par un seul boitier électronique de commande (3).

Un montage en série tel que ci-dessus décrit est avantageux lorsqu'on souhaite atteindre des cibles se trouvant à des endroits spatialement différents.

Le système matriciel autonome piloté conforme à l'invention a plusieurs avantages. Il est apte à distribuer de façon autonome des substances actives en quantité contrôlé et modulable uniquement aux moments opportuns et précis en conférant à un traitement donné une meilleure efficacité. En effet, grâce à la programmation de la carte électronique qui pilote l'alimentation électrique des composants électriques par le générateur, l'utilisateur active volontairement le système pour qu'il enclenche le relargage de la substance active selon un profil de référence. De fait, il permet d'offrir la possibilité de valoriser l'efficacité intrinsèque d'un actif efficace à température ambiante plutôt par contact et qui, par chauffage, le devient par diffusion par volatilité. Le système conforme à l'invention permet également de vider la matrice active de la quasi totalité de sa substance active contrairement aux dispositifs classiques à diffusion passive à la température ambiante. En fonction des polymères utilisés, on peut agir sur le mode de fonctionnement du système ce qui représente une grande flexibilité et une forte adaptabilité d'application. Enfin, lorsqu'il est monté en série, ou que les matrices sont multiples, le système matriciel piloté est capable de distribuer simultanément une ou plusieurs substances actives différentes pour plusieurs cibles identiques et/ou différentes même si celles-ci sont spatialement éloignées les unes par rapport aux autres. Dans ce cas, une matrice active est stimulée indépendamment l'une par rapport à autre pour une efficacité meilleure.

Les exemples ci-après servent à illustrer le système matriciel selon l'invention, sans en restreindre la portée.

### Exemple 1 : système matriciel autonome piloté en matrice polymères thermodurcissables.

Préalablement, les expériences menées dans les laboratoires de la Demanderesse ont permis d'identifier l'huile essentielle de menthe poivrée (HEMP) comme substance active ayant un effet répulsif significatif sur les mouches domestiques (*Musca domestica*)*.* Pour ce faire, un olfactomètre à trois entrées a démontré les quantités significativement suffisantes d'HEMP pour obtenir un effet répulsif vis-à-vis des mouches. Le tableau 1 ci-après consigne les résultats obtenus.

**Tableau 1 : Effet répulsif de l'HEMP vis-à-vis des mouches.**

| | | | | |
|---|---|---|---|---|
| Débit moyen d'HEMP (mg/heure) | 0 | 5,2 | 25,8 | 48,2 |
| Pourcentage de temps moyen passé par les mouches en présence d'HEMP | 55% | 47% | 5,6% | 2,1% |

### Matériel :

On dispose de :
- un réacteur cylindrique ouvert de 1 litre muni d'un agitateur métallique se terminant par un disque de 6 cm de diamètre, l'agitateur étant actionné par un moteur à variation de vitesse,
- un transformateur électrique de 24V DC, 2,5 A et 60 W assurant l'alimentation électrique du système ;
- 2 capteurs thermiques de type PT100 ;
- un fil chauffant gainé dont la résistance est de 80 Ω/m pour une longueur totale de 25 cm. Les deux extrémités du fil chauffant sont directement reliées au générateur électrique ;
- un boitier électronique dans lequel est logée une carte électronique supportant un calculateur (microcontrôleur) qui gère à la fois les séquences de chauffage, la puissance délivrée par le transformateur comprise entre 0,1 et 60W et les interfaces électriques. Les capteurs et le fil chauffant sont reliés au boitier électronique via un bornier. Le microcontrôleur est configuré par ordinateur au moyen d'une connexion USB afin que l'alimentation de la matrice active s'effectue par les séquences d'activation programmées.
- un moule en polypropylène dont l'empreinte est une forme cylindrique ouverte de 10 cm de diamètre pour 2 cm d'épaisseur.

Pour fabriquer la matrice active, on dispose des intrants suivants :
- une résine polyamine à chaine courte de PM= 178,28 et de fonctionnalité 2 commercialisée sous la marque XL 1701® ;
- une résine polyol de PM=4800 et de fonctionnalité 3, commercialisée sous la marque G48® ;
- un prépolymère de diisocyanate de toluène (TDI) dont le pourcentage NCO est de 6,1, de fonctionnalité 2, commercialisé sous la marque LU-T 95® ;
- un prépolymère de MDI (diisocyanate de diphénylméthylène) dont le pourcentage NCO est de 24,5 de fonctionnalité 2 commercialisé sous la marque SUPRASEC 2029® ;
- de l'huile essentielle de menthe poivrée (HEMP) comme substance active à action répulsive contre les mouches.
- du solvant qui est le 2-éthylhexyldiphényl phosphate commercialisé sous la marque SANTICIZER® 141 ;
- un colorant rouge sous forme de poudres fines commercialisé sous la marque Rouge au gras W320®.

Toutes les opérations suivantes sont réalisées à la température ambiante.

### Préparation de la composition liquide contenant l'HEMP:

On introduit dans un bêcher, 18,3 g d'HEMP, 51,2 g de SANTICIZER® 141, 0,1 g de colorant, puis on mélange le tout par agitation faible. Après 5 mn d'agitation, on obtient une composition active sous forme d'un mélange liquide homogène.

### Préparation du mélange liquide polyol :

On introduit successivement dans le réacteur, 13,2 g de résine XL1701® sous forme liquide et la composition active liquide obtenue ci-dessus. On mélange le tout par agitation à 1300 tr/mn pendant 30 secondes. On obtient un mélange liquide homogène appelé « complexe de phase polyol ».

On ajoute dans le complexe de phase polyol obtenu, 100 g de diisocyanate LU-T 95® puis on agite à 1300 tr/mn pendant 30 secondes. On obtient un mélange liquide homogène de polyuréthane prêt à être coulé.

### Mise en forme de la matrice active en polyuréthane coulé :

On coule dans le moule cylindrique ci-dessus décrit le mélange liquide homogène de polyuréthane. Lorsque ledit mélange remplit le moule jusqu'à mi-hauteur, on y dispose le fil chauffant en zigzag et dont les deux extrémités dépassent pour pouvoir les relier au générateur. Au même moment, on répartit également les 2 capteurs thermiques sur la matrice en cours de polymérisation. Ensuite, on coule le reste du mélange liquide de polyuréthane jusqu'aux dimensions souhaitées. Le fil chauffant ainsi que les capteurs thermiques sont ainsi encastrés dans la matrice active. Puis, on laisse se finaliser la polymérisation. On démoule la pièce au bout de 3 minutes environ. On obtient une pièce cylindrique de 10 cm de diamètre pour 2 cm d'épaisseur en matrice polyuréthane chargée à 10% d'HEMP en poids par rapport au poids total de la pièce. Les deux extrémités du fil chauffant sont ensuite reliées au transformateur électrique lequel est relié au boitier électronique.

Il est à noter que la température de dégradation structurelle irréversible de la matrice en polyuréthane telle qu'elle est formulée pour cette expérience est environ 170°C et que le point d'ébullition de l'HEMP est d'environ 210°C.

### Activation par chauffage de la matrice chargée d'HEMP :

Afin d'obtenir les quantités d'HEMP nécessaires pour avoir un effet répulsif efficace contre les mouches, on programme la carte électronique de la façon suivante :
- coupure systématique de la chauffe donc du générateur lorsque la température intra-matricielle relevée par les capteurs thermiques est de 150°C ;
- activation de la chauffe respectivement pendant 13, 7 et 9 minutes ce qui correspond à trois séquences d'activation ;
- réglage de la chauffe pour 110 séquences d'activation pour que la quantité résiduelle d'HEMP à la fin de la dernière activation soit inférieure à 10% en poids par rapport à la quantité initialement incorporée ;
- réglage de la puissance du transformateur à 30 watts, donc de l'intensité de la chauffe ;
- inactivation de la chauffe tant que la température intra-matricielle mesurée est supérieure à 22°C afin de laisser la matrice active reprendre son comportement initial.

Un interrupteur permet de couper l'alimentation en courant du système.

La programmation définie ci-dessus permet d'obtenir un profil de relargage de référence qui peut être optimisé par l'ajout d'autres collecteurs de signaux tels que des détecteurs de mouvement ou de présence de mouches placés à proximité des matrices actives.

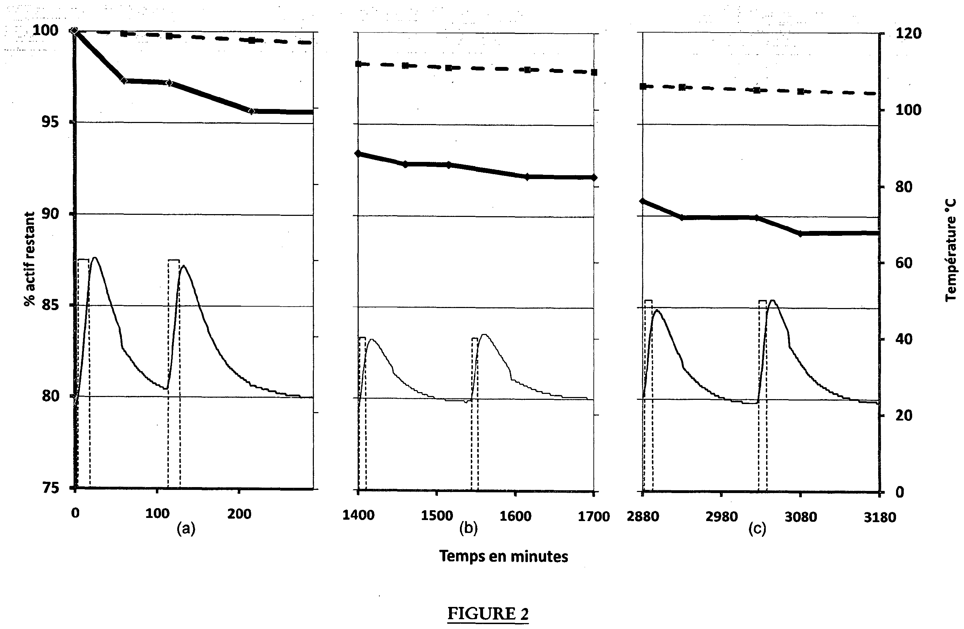

La figure 2 représente le profil de relargage de référence de l'HEMP. Sont représentées les courbes de relargage par diffusion passive de l'HEMP à partir d'une matrice en polyuréthane non chauffée placée à la température ambiante (courbes en traits discontinus) et les courbes de relargage à partir d'une matrice en polyuréthane dans laquelle est encastré un fil chauffant (traits épais continus). La courbe en traits fins continus présente le relevé de température intra-matricielle des pièces chauffées. Les expériences décrites ci-dessus sont menées sur 3 matrices différentes (voir tableau 2).

Les 3 durées de chauffe sont respectivement de (a) 13, (b) 7 et (c) 9 minutes. La matrice chargée d'HEMP est pesée après chaque chauffage afin de déterminer la quantité effective d'HEMP relarguée. Après chaque chauffage, on laisse la matrice au repos, à la température ambiante, pendant une durée minimum de 40 minutes pour que la température baisse jusqu'à 35°C et 22°C. Parallèlement, une cinétique de relargage est effectuée sur une matrice en polyuréthane identique et chargée à 10% d'HEMP en poids, mais dépourvue d'élément chauffant. Celle-ci libère faiblement par diffusion passive l'HEMP dont la quantité relarguée est également mesurée par pesée différentielle.

Les températures intra-matricielles mesurées sont de 60°C, 40°C et 50°C respectivement pour les durées de chauffe de 13, 7 et 9 minutes.

Le tableau 2 ci-après récapitule la quantité d'HEMP relarguée à partir de 3 matrices en polyuréthane. Chaque matrice pèse environ 100g pour une quantité d'HEMP de 10 g soit 10% du poids total de la matrice.

**Tableau 2 : quantités moyennes d'HEMP relarguée à partir de 3 matrices en polyuréthane coulé.**

| Matrices en polyuréthane | Débit de relaraage de l'HEMP en fonction de la durée d'activation | | | |
|---|---|---|---|---|
| | **Sans activation** | **7 minutes d'activation** | **9 minutes d'activation** | **13 minutes d'activation** |
| Matrice 1 (M1) | 10 mg/heure | 45 mg/heure | 65 mg/heure | 110 mg/heure |
| Matrice 2 (M2) | 9 mg/heure | 35 mg/heure | 50 mg/heure | 55 mg/heure |
| Matrice 3 (M3) | 6 mg/heure | 25 mg/heure | 40 mg/heure | 45 mg/heure |

### Composition de la matrice 1 :

La solution liquide constituant la phase polyol comprend : 13,2g de XL1701®, 18,3g d'HEMP, 0,1g de colorant, 51,2g de SANTICIZER 141® ;
La solution liquide de la phase isocyanate est constituée de 100g de LUT-95®.

### Composition de la matrice 2 :

La solution liquide constituant la phase polyol comprend : 81,24g de G48®, 10g d'HEMP, 0,1g de colorant et 5,12g de SANTICIZER 141® ;
La solution liquide de la phase isocyanate est constituée de 8,71g de SS2029®

### Composition de la matrice 3 :

La solution liquide constituant la phase polyol comprend : 13,2g de XL1701®, 12,58g d'HEMP et 0,1g de colorant rouge ;
La solution liquide de la phase isocyanate est constituée de 100g de LUT-95®.

On note que si l'on souhaite obtenir un effet répulsif de 5,6% ce qui correspond à un débit d'HEMP de 25,8 mg/h, le système conforme à l'invention permet d'utiliser plusieurs possibilités notamment la matrice M2/7 minutes ou encore la matrice M3/7 minutes. De même, pour obtenir un effet répulsif de 2,1%, on peut utiliser notamment la matrice M1/9 minutes, la matrice M2/9 minutes ou encore M2/13 minutes.

On constate également que le système conforme à l'invention permet d'obtenir un débit d'HEMP contrôlé uniquement à des moments précis et en quantité suffisante pour repousser significativement les mouches. Après 110 séquences d'activation d'une durée de 13 minutes, la quantité d'HEMP résiduelle mesurée dans la matrice M1 est de 3% par rapport à la quantité initialement incorporée.

Exemple 2 : système matriciel autonome piloté dont la matrice est en polymères thermoplastiques d'origine fossile.

On dispose des mêmes matériels que dans l'exemple 1 (transformateur électrique, capteurs thermiques, carte électronique programmée).

On dispose d'une presse à injecter de la marque ARBURG sur laquelle est monté un moule portant l'empreinte d'un médaillon de forme ovale.

On dispose d'une plaque chauffante Heidolph (MR HEI Standard) ayant une puissance surfacique de 4,84 watts/cm². Ladite plaque chauffante est encastrée dans le médaillon ce qui permet de chauffer uniformément ledit médaillon.

On dispose des granulés de copolymères d'éthylène et d'acétate de vinyle (EVA) commercialisés sous la marque EVA538® et EVA461® ainsi que du citriodiol comme substance insectifuge.

### Préparation de la composition liquide contenant la substance active :

On mélange dans un bécher 13,5 g de citriodiol et 2 g d'huile de coprah. Après 5 mn d'agitation faible, on obtient un mélange liquide homogène.

### Incorporation de la composition liquide de citriodiol dans les granulés d'EVA :

- On introduit 67,5 g de granulés d'EVA538® dans le réacteur en verre préalablement préchauffé à 70°C ;
- On introduit par goutte à goutte la composition liquide ;
- On agite le tout jusqu'à ce que tout le liquide soit incorporé dans les granulés ;
- On refroidit le réacteur à 40°C puis on introduit 17 g de granulés d'EVA461® tout en agitant faiblement ;
- On refroidit le tout à 20°C puis on récupère les granulés chargés à 13,5% de citriodiol.

### Mise en forme de la matrice active :

Les granulés d'EVA chargés de citriodiol obtenus ci-dessus sont mis en forme de médaillon ovale de 8 mm d'épaisseur, 20 cm² de face pour environ 10g.

Deux capteurs thermiques sont insérés dans le médaillon obtenu ci-dessus afin de mesurer la température intra-matricielle au cours de la chauffe.

### Activation par chauffage du médaillon chargé de citriodiol :

On programme la carte électronique de la manière suivante :
- coupure systématique de la chauffe donc du générateur lorsque la température intra-matricielle relevée par les capteurs thermiques est de 60°C ;
- activation de la chauffe toutes les 10 minutes suivi d'un temps de pause de 20 minutes ce qui correspond à une séquence d'activation d'une durée totale de 30 minutes ;
- réglage de la chauffe pour 36 séquences d'activation ;
- réglage de la puissance du transformateur à 30 watts, donc de l'intensité de la chauffe ;
- inactivation de la chauffe tant que la température intra-matricielle mesurée est supérieure à 22°C afin de laisser la matrice active reprendre son comportement initial.

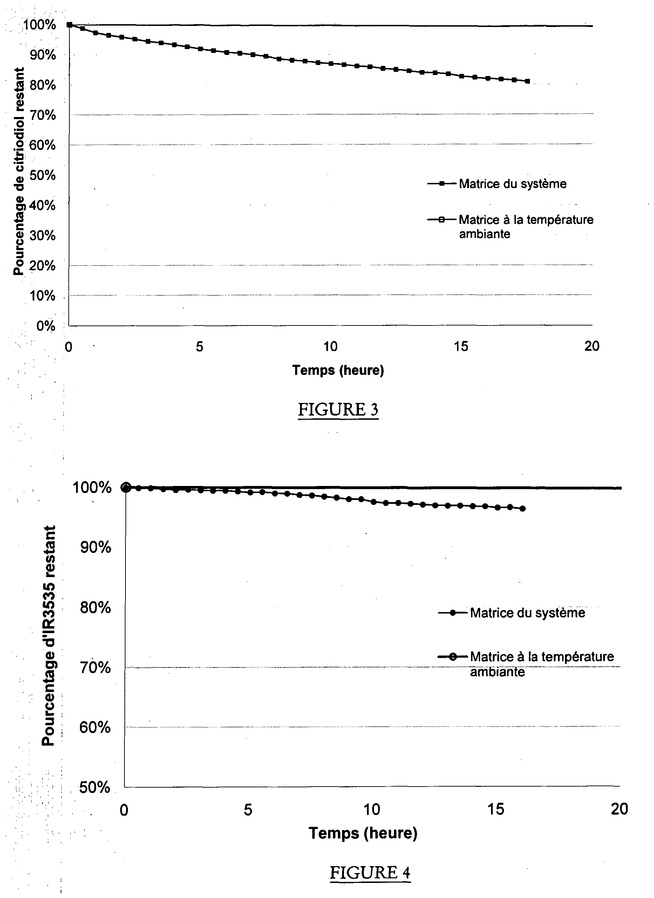

La figure 3 représente le profil de relargage de référence du citriodiol. Sont représentées les courbes de relargage par diffusion passive du citriodiol à partir d'une matrice en EVA non chauffée et placée à la température ambiante (courbe en trait continu) et la courbe de relargage correspondant à une matrice identique en EVA dans laquelle est encastrée une plaque chauffante (courbe en trait continu sur laquelle chaque activation est matérialisée par un carrée) conforme au système objet de l'invention. Les activations séquentielles et successives ont été menées pendant 18 heures à raison de 10 minutes de chauffe suivies de 20 minutes de pause. La température intra-matricielle relevée est de 45°C.

Le système conforme à l'invention permet ainsi de relarguer jusqu'à 20% en poids du citriodiol initialement incorporé au bout de 18 heures. En revanche, la même matrice n'en relargue pratiquement pas à la température ambiante. Par ailleurs, le système permet de relarguer une quantité moyenne de citriodiol de 5 mg/heure uniquement aux moments précis de l'activation.

Enfin, après 185 séquences de chauffe, et à la fin de la dernière chauffe, la quantité résiduelle de citriodiol mesurée est de 9% en poids par rapport à la quantité initialement incorporée.

Exemple 3 : système matriciel autonome piloté en matrice polymères thermodurcissables dont la substance active est l'IR3535®

On dispose des mêmes matériels que dans l'exemple 1, à l'exception du fil chauffant qui est remplacé par une plaque chauffante comme dans l'exemple 2.

Pour la matrice, on dispose des mêmes intrants que dans l'exemple 1. L'HEMP est remplacée par l'insecticide (IR3535®) qui représente 30% en poids par rapport au poids total de la matrice.

La carte électronique est programmée comme dans l'exemple 2 sauf que la chauffe est systématiquement coupée lorsque la température intra-matricielle est de 150°C.

La figure 4 représente le profil de relargage de référence de l'IR3535® qui est une substance non volatile à la température ambiante. Sont représentées les courbes de relargage par diffusion passive de l'IR3535® à partir d'une matrice en polyuréthane coulé non chauffée et placée à la température ambiante (courbe en trait épais continu) et la courbe de relargage correspondant à une matrice identique en polyuréthane coulé dans laquelle est encastrée une plaque chauffante (trait continu sur laquelle chaque activation est matérialisée par un rond) conforme au système objet de l'invention. Les activations séquentielles et successives ont été menées pendant 18 heures à raison de 10 minutes de chauffe suivies de 20 minutes de pause. La température intra-matricielle relevée est de 45°C.

Le système conforme à l'invention permet ainsi de relarguer jusqu'à 3% en poids d'IR3535® initialement incorporé au bout de 18 heures. En revanche, la même matrice n'en relargue absolument pas à la température ambiante même au bout de 20 heures. Par ailleurs, le système permet de relarguer une quantité moyenne d'IR3535® de 3,5 mg/heure uniquement aux moments précis de l'activation.

Après 590 séquences de chauffe, et à la fin de la dernière chauffe, la quantité résiduelle d'IR3535® mesurée est de 9,5% en poids par rapport à la quantité initialement incorporée.

## Revendications

1. Système matriciel (1) autonome et piloté pour la distribution contrôlée et modulable uniquement à des moments opportuns et précis d'au moins une substance active, ***caractérisé en ce qu***'il comprend :
- une matrice polymérique non stratifiée (2) chargée en ladite substance active, ladite matrice comportant au moins une sonde thermique (7),
- au moins un élément chauffant (4) encastré dans ladite matrice (2) chargée, ledit élément chauffant (4) étant apte à chauffer de façon homogène l'intégralité de ladite matrice (2) de manière à dilater temporairement le réseau polymérique pour enclencher le relargage de ladite substance active, et
- un générateur (5) de courant électrique relié à une carte électronique de commande, elle-même reliée audit élément chauffant (4), dont l'alimentation en courant électrique par ledit générateur (5) est pilotée par ladite carte logée dans un boitier électronique (3), ladite carte étant reliée, en outre, à ladite au moins sonde thermique (7).

2. Système selon la revendication 1, ***caractérisé en ce que*** la programmation de ladite carte consiste à faire varier trois paramètres essentiels que sont l'intensité de chauffe, la durée de chauffe et la fréquence de chauffe, lesdits paramètres étant dépendants ou non les uns par rapport aux autres.

3. Système selon l'une des revendications 1 et 2, ***caractérisé en ce que*** ladite carte exécute les fonctions choisies parmi :
- gestion de la mise en marche et de l'arrêt de l'alimentation en courant électrique de(s) l'élément(s) chauffant(s) (4) par le générateur (5) ;
- réglage de la puissance développée par ledit générateur (5) ;
- réglage de la durée, l'intensité et la fréquence de la chauffe ;
- gestion des séquences d'activation ;
- gestion de la durée totale d'un traitement ;
- gestion, le cas échéant, de la mise en marche, l'arrêt et la puissance d'un ventilateur.

4. Système selon l'une des revendications précédentes, ***caractérisé en ce que*** la chauffe est systématiquement coupée soit lorsque la température intra-matricielle est de 2°C à 5°C en dessous des températures de dénaturation de la substance active et/ou de la matrice soit lorsqu'elle est égale à la température de consigne préalablement indiquée lors de la programmation.

5. Système selon l'une des revendications 1 et 4, ***caractérisé en ce que*** la chauffe s'effectue de façon séquentielle ou en continue jusqu'à ce que la quantité résiduelle de la substance active dans la matrice active (2) soit inférieure à 10%, préférentiellement entre 0,1% et 5% en poids par rapport à la quantité initialement incorporée.

6. Système selon la revendication 5, ***caractérisé en ce que*** la durée d'une chauffe varie entre 10 secondes et plusieurs heures.

7. Système selon la revendication 1, ***caractérisé en ce que*** la mise en marche du système (1) se fait de manière automatique ou au moyen des enclencheurs manuels comme les interrupteurs et analogues.

8. Système selon l'une des revendications 1 et 2, ***caractérisé en ce que*** ladite carte électronique est reliée à un collecteur de signaux apte à transformer les grandeurs physico-chimiques en signaux électriques, lesdites grandeurs physico-chimiques sont choisies parmi la température, la pression, le pH, l'air, la fumée, l'humidité, le bruit, le mouvement, la luminosité ou l'association de celles-ci.

9. Système selon la revendication 8, ***caractérisé en ce que*** ledit collecteur est choisi parmi un capteur, une sonde, un détecteur ou l'association de ceux-ci.

10. Système selon la revendication 1, ***caractérisé en ce que*** l'élément chauffant (4) est surmoulé directement dans la matrice (2) ou s'emboite avec celle-ci.

11. Système selon l'une des revendications 1, 4 et 5, ***caractérisé en ce que*** la substance active représente entre 0,1% et 50% en poids par rapport au poids total de la matrice active (2).

12. Système selon la revendication 11, ***caractérisé en ce que*** ladite substance active a une fonction choisie parmi une fonction attractive, répulsive, insecticide, insectifuge, fongicide, larvicide, bactéricide, rodenticide, parfumante, désodorisante, cosmétique, ou phytosanitaire.

13. Système selon la revendication 12, ***caractérisé en ce que*** la substance insecticide/insectifuge est choisie parmi les composés de la famille des pyréthrinoides et leurs dérivés, les pyréthrines, le DEET (N, N-diéthyl-3-méthylbenzamide), l'Icaridine®, l'éthyl-3-acetyl(butyl)amino-propanoate (IR3535®), les organochlorés, les organophosphorés, les organophosphates, les carbamates, les néonicotinoïdes, les sulfones et sulfonates, les formamidines, les benzoylurées, les huiles essentielles, les extraits végétaux, les hydrocarbures terpéniques et leurs dérivés, les huiles végétales ou le mélange de ceux-ci.

14. Système selon l'une des revendications 1, 4, 5, 10 et 11, ***caractérisé en ce que*** la matrice active (2) est fabriquée à partir de polymères thermoplastiques synthétiques ou biousourcés, biodégradables ou non ou encore de polymères thermodurcissables.

15. Procédé pour traiter efficacement des cibles, constituée(s) des insectes ou une zone délimitée dans un environnement ouvert ou fermé, se trouvant au même endroit ou à des endroits spatialement différents au moyen d'au moins une substance active, dont la diffusion séquentielle ou en continue d'une quantité contrôlée préalablement déterminée uniquement à des moments opportuns et précis, ledit procédé consiste à utiliser le système selon la revendication 1.

16. Procédé selon la revendication 15, ***caractérisé en ce que*** lorsque les matrices actives (2) sont multiples et réparties à différents endroits ou qu'elles sont montées en série, chaque l'élément chauffant (4) de chacune des matrices (2) est relié à une carte électronique de commande unique et commune et, est disposée au sein d'un poste central unique faisant office de boitier électronique (3), à partir duquel ladite carte pilote, de manière individuelle, l'activation de chacun des éléments chauffants (4), en fonction des données récoltées en provenance des collecteurs, ladite carte étant elle-même reliée soit par un câble électrique porteur, lui même relié à un générateur (5) de courant, soit reliée à une pile.

## Patentansprüche

1. Autonomes und kontrolliertes Matrixsystem (1) zur kontrollierten und modularen Verteilung, nur zu geeigneten und genauen Zeiten, von mindestens einem Wirkstoff, **dadurch gekennzeichnet, dass** es Folgendes umfasst:
- eine unlaminierte polymere Matrix (2), die mit dem Wirkstoff beladen ist, wobei die Matrix mindestens einen Temperaturfühler (7) enthält,
- mindestens ein Heizelement (4), das in die beladene Matrix (2) eingebettet ist, wobei das Heizelement (4) in der Lage ist, die gesamte Matrix (2) homogen zu erwärmen, um so das Polymernetzwerk vorübergehend zu erweitern, um die Freisetzung des Wirkstoffs zu aktivieren, und
- einen elektrischen Stromgenerator (5), der mit einer elektronischen Steuerplatine verbunden ist, die selbst mit dem Heizelement (4) verbunden ist, dessen Stromversorgung mittels Generator (5) durch die in einem Schaltkasten (3) untergebrachte Platine gesteuert wird, wobei die Platine zusätzlich mit dem mindestens einen Temperaturfühler (7) verbunden ist.

2. System nach Anspruch 1, **dadurch gekennzeichnet, dass** die Programmierung der Platine darin besteht, drei wesentliche Parameter zu variieren, nämlich die Heizintensität, die Heizdauer und die Heizfrequenz, wobei die Parameter voneinander abhängig sind oder nicht.

3. System nach einem der Ansprüche 1 und 2, **dadurch gekennzeichnet, dass** die Platine die Funktionen ausführt, ausgewählt aus:
- Steuerung des Ein- und Ausschaltvorgangs der Stromversorgung des/der Heizelemente(s) (4) durch den Generator (5);
- Einstellung der von dem Generator (5) erzeugten Leistung;
- Einstellung von Dauer, Intensität und Frequenz der Heizung;
- Steuerung von Aktivierungssequenzen;
- Steuerung der Gesamtdauer einer Behandlung;
- gegebenenfalls Steuerung des Ein- und Ausschaltvorgangs und der Leistung eines Lüfters.

4. System nach einem der vorhergehenden Ansprüche, **dadurch gekennzeichnet, dass** die Heizung systematisch abgeschaltet wird, entweder wenn die Innenmatrix-Temperatur 2 °C bis 5 °C unter den Denaturierungstemperaturen des Wirkstoffs und/oder der Matrix liegt oder wenn sie der zuvor bei der Programmierung angegebenen Solltemperatur entspricht.

5. System nach einem der Ansprüche 1 und 4, **dadurch gekennzeichnet, dass** die Erwärmung sequentiell oder kontinuierlich durchgeführt wird, bis die Restmenge des Wirkstoffs in der aktiven Matrix (2) weniger als 10 Gew.-% beträgt, vorzugsweise zwischen 0,1 und 5 Gew.-%, und zwar bezogen auf die ursprünglich eingebrachte Menge.

6. System nach Anspruch 5, **dadurch gekennzeichnet, dass** die Dauer einer Erwärmung zwischen 10 Sekunden und mehreren Stunden variiert.

7. System nach Anspruch 1, **dadurch gekennzeichnet, dass** das System (1) automatisch oder mittels manueller Auslöser, wie Schalter und dergleichen, eingeschaltet wird.

8. System nach einem der Ansprüche 1 und 2, **dadurch gekennzeichnet, dass** die Platine mit einer Signalsenke verbunden ist, die in der Lage ist, physikalisch-chemische Größen in elektrische Signale umzuwandeln, wobei die physikalisch-chemischen Größen aus Temperatur, Druck, pH-Wert, Luft, Rauch, Feuchtigkeit, Rauschen, Bewegung, Helligkeit oder einer Kombination derselben ausgewählt sind.

9. System nach Anspruch 8, **dadurch gekennzeichnet, dass** die Signalsenke aus einem Sensor, einer Sonde, einem Detektor oder einer Kombination davon ausgewählt ist.

10. System nach Anspruch 1, **dadurch gekennzeichnet, dass** das Heizelement (4) direkt in die Matrix (2) geformt ist oder sich mit dieser zusammenschließt.

11. System nach einem der Ansprüche 1, 4 und 5, **dadurch gekennzeichnet, dass** der Wirkstoff zwischen 0,1 und 50 Gew.-%, bezogen auf das Gesamtgewicht der aktiven Matrix (2), beträgt.

12. System nach Anspruch 11, **dadurch gekennzeichnet, dass** der Wirkstoff eine Funktion aufweist, die aus einer anziehenden, abstoßenden, insektenbekämpfenden, insektenabweisenden, pilztötenden, larventötenden, keimabtötenden, nagetierbekämpfenden, duftstoffhaltigen, deodorierenden, kosmetischen oder pflanzengesundheitlichen Funktion ausgewählt ist.

13. System nach Anspruch 12, **dadurch gekennzeichnet, dass** die insektenbekämpfende/insektenabweisende Substanz aus den Verbindungen der Pyrethroid-Familie und deren Derivaten, Pyrethrinen, DEET (N, N-Diethyl-3-methylbenzamid), Icaridin®, Ethyl-3-acetyl(butyl)amino-propanoat (IR3535®), Organochlorverbindungen, Organophosphor, Organophosphaten, Carbamaten, Neonicotinoiden, Sulfonen und Sulfonaten, Formamidinen, Benzoylharnstoffen, ätherischen Ölen, Pflanzenextrakten, terpenischen Kohlenwasserstoffen und deren Derivaten, pflanzlichen Ölen oder einer Mischung davon ausgewählt ist.

14. System nach einem der Ansprüche 1, 4, 5, 10 und 11, **dadurch gekennzeichnet, dass** die aktive Matrix (2) aus synthetischen oder biobasierten thermoplastischen Polymeren, biologisch abbaubar oder nicht abbaubar, oder aus duroplastischen Polymeren hergestellt ist.

15. Verfahren zur effektiven Behandlung von Zielen, bestehend aus Insekten oder abgegrenzten Bereichen in einer offenen oder geschlossenen Umgebung, die sich an denselben oder räumlich verschiedenen Orten mittels mindestens eines Wirkstoffsbefinden, dessen sequentielle oder kontinuierliche Verteilung einer zuvor bestimmten kontrollierten Menge nur zu angemessenen und genauen Zeiten erfolgt, wobei das besagte Verfahren die Verwendung des Systems nach Anspruch 1 umfasst.

16. Verfahren nach Anspruch 15, **dadurch gekennzeichnet, dass**, wenn die aktiven Matrizen (2) mehrfach und an verschiedenen Stellen verteilt oder in Reihe geschaltet sind, jedes der Heizelemente (4) von jeder der Matrizen (2) mit einer einzigen gemeinsamen elektronischen Steuerplatine verbunden ist und innerhalb einer einzigen Zentralstation angeordnet ist, die als Schaltkasten (3) fungiert, von dem aus die Platine die Aktivierung jedes der Heizelemente (4) einzeln steuert, und zwar gemäß den von den Signalsenken gesammelten Daten, wobei die Platine selbst entweder durch eine Batterie verbunden ist, oder durch ein Elektro-Tragkabel, das wiederum mit einem Generator (5) verbunden ist.

## Claims

1. Controlled stand-alone matrix system (1) for the controlled and adjustable distribution of at least one active substance only at precise, appropriate times, **characterized in that** it comprises:
- a non-laminated polymer matrix (2) loaded with said active substance, said matrix comprising at least one temperature probe (7),
- at least one heating element (4) embedded in said loaded matrix (2), said heating element (4) being capable of uniformly heating the entire matrix (2) so as to temporarily expand the polymer network in order to trigger the release of said active substance, and
- an electricity generator (5) which is connected to an electronic control board, which is in turn connected to said heating element (4), the electricity supply of which by said generator (5) is controlled by said board, which is accommodated in an electronic box (3), said board also being connected to said at least one temperature probe (7).

2. System according to claim 1, **characterized in that** the programming of said board involves varying three essential parameters of heating intensity, heating duration and heating frequency, said parameters being dependent on or independent of one another.

3. System according to either claim 1 or claim 2, **characterized in that** said board executes the functions selected from:
- management of the starting and stopping of the electricity supply to the heating element(s) (4) by the generator (5);
- adjustment of the power developed by said generator (5);
- adjustment of the duration, intensity and frequency of the heating;
- management of the activation sequences;
- management of the total duration of a treatment;
- management, if necessary, of the starting, stopping and power of a fan.

4. System according to any of the preceding claims, **characterized in that** the heating is systematically stopped either when the intra-matrix temperature is from 2°C to 5°C below the denaturing temperature of the active substance and/or of the matrix, or when said intra-matrix temperature is equal to the setpoint temperature previously indicated during the programming.

5. System according to either claim 1 or claim 4, **characterized in that** the heating is carried out sequentially or continuously until the residual amount of the active substance in the active matrix (2) is less than 10 wt.%, preferably between 0.1 wt.% and 5 wt.%, based on the amount initially incorporated.

6. System according to claim 5, **characterized in that** the duration of heating varies between 10 seconds and several hours.

7. System according to claim 1, **characterized in that** the system (1) starts automatically or by means of manual actuators such as switches and the like.

8. System according to either claim 1 or claim 2, **characterized in that** said electronic board is connected to a signal collector capable of converting physicochemical quantities into electrical signals, said physicochemical quantities being selected from temperature, pressure, pH, air, smoke, humidity, noise, motion, luminosity or a combination thereof.

9. System according to claim 8, **characterized in that** said collector is selected from a sensor, a probe, a detector or a combination thereof.

10. System according to claim 1, **characterized in that** the heating element (4) is over-molded directly into the matrix (2) or fits inside said matrix.

11. System according to any of claims 1, 4 and 5, **characterized in that** the active substance represents between 0.1 wt.% and 50 wt.% of the total weight of the active matrix (2).

12. System according to claim 11, **characterized in that** said active substance has a function selected from the functions of an attractant, repellent, insecticide, insect repellent, fungicide, larvicide, bactericide, rodenticide, fragrance, deodorant, cosmetic or phytosanitary product.

13. System according to claim 12, **characterized in that** the insecticide/insect repellent substance is selected from compounds of the pyrethroid family and the derivatives thereof, pyrethrins, DEET (N,N-diethyl-3-methylbenzamide), Icaridine®, ethyl-3-acetyl(butyl)amino-propanoate (IR3535®), organochlorides, organophosphorates, organophosphates, carbamates, neonicotinoids, sulfones and sulfonates, formamidines, benzoylureas, essential oils, plant extracts, terpene hydrocarbons and the derivatives thereof, vegetable oils or a mixture thereof.

14. System according to any of claims 1, 4, 5, 10 and 11, **characterized in that** the active matrix (2) is produced from synthetic or bio-based thermoplastic polymers, whether biodegradable or not, or thermosetting polymers.

15. Method for effectively treating targets, in the form of insects or a delimited region in an open or closed environment, which are located in the same place or in spatially different places, by means of at least one active substance, a predetermined, controlled amount of which is diffused sequentially or continuously only at precise, appropriate times, said method involving using the system according to claim 1.

16. Method according to claim 15, **characterized in that** when there are multiple active matrices (2) distributed in different places or installed in series, each heating element (4) of each of the matrices (2) is connected to a single common electronic control board and arranged in a single central station acting as an electronic box (3) from which said board controls the activation of each of the heating elements (4) individually, depending on the data collected from the collectors, said board either being itself connected by a carrier electric cable, which is in turn connected to a current generator (5), or being connected to a battery.
